# EUROPEAN PATENT SPECIFICATION

(11) **EP 0 418 945 B1**
(45) Date of publication and mention of the grant of the patent: **09.11.1994**
(21) Application number: 90202264.9
(22) Date of filing: 22.08.1990
(51) Int. Cl.: C12N 9/10, C08B 31/00, A23L 1/105, A23K 1/00, C12N 15/54

(54) **A branching enzyme and its use**
Verzweigungsenzym und dessen Verwendung
Enzyme branchante et son utilisation

(30) Priority: 23.08.1989 NL 8902128
(43) Date of publication of application: 27.03.1991
(73) Proprietor: Coöperatieve Verkoop- en Productievereniging van Aardappelmeel en Derivaten 'AVEBE' B.A., NL-9641 JA Veendam (NL)
(72) Inventor: Kiel, Jan Andries Kornelis Willem, NL 9721 LA Groningen (NL); Boels, Johannes Martinus, NL 9791 GD Ten Boer (NL); Beldman, Gerrit, NL 6708 ML Wageningen (NL); Venema, Gerhardus, NL 9751 NN Haren (NL)
(74) Representative: Smulders, Theodorus A.H.J., Ir.

(56) References cited:
- EP-A- 0 057 976
- FR-A- 2 499 588
- THE JOURNAL OF BIOLOGICAL CHEMISTRY, vol. 261, no. 19, 5th July 1986, pages 8738-8743; P.A. BAECKER et al.: "Biosynthesis of bacterial glycogen"
- GENE, vol. 78, no. 1, 15th May 1989, pages 9-17; J.A.K.W. KIEL et al.: "Cloning and expression of the branching enzyme gene (glgB) from the cyanobacterium Synechococcus sp. PCC7942 in Escherichia coli"

## Description

### Field of the invention

This invention is in the fields of recombinant DNA technology, microbiology, enzymology and starch and food technology. The invention relates to a specific enzyme capable of being used, e.g., for the preparation of foods, and relates to the enzyme-encoding gene cloned by the inventors and capable of being used for transformation of an organism suitable for production of the enzyme.

More in particular, the invention relates to a branching enzyme of microbial origin, to a use of this branching enzyme for the modification of a starch-like material, e.g., within the scope of a process for the preparation or manufacture of human or animal foods, as well as to a recombinant polynucleotide (especially recombinant DNA, e.g., in the form of a chimeric plasmid) comprising the genetic information coding for the branching enzyme.

### Background of the invention

Different organisms (a term used here in its most extended meaning and therefore including both bacteria and other small organisms such as fungi, yeasts and algae) possess an enzyme involved in the biosynthesis of glycogen and known as branching enzyme. This enzyme is a transferase leading to the formation of a(1fi6) branches in starch-like substrates such as amylose, amylopectin, starch, dextrin and polysaccharides derived therefrom and composed of α-D-glucose. The systematic name of this enzyme is 1,4-α-D-glucan:1,4-α-D-glucan 6-α-D-(1,4-α-D-glucano)-transferase, or EC 2.4.1.18. Glycogen is a polyglucose material related to the vegetable starch component amylopectin and serving in animals and different microorganisms as a carbon and energy-storage material. Like amylopectin, glycogen is a branched polyglucose material, but the branching frequency is higher in glycogen than in amylopectin. The branching enzyme is necessary to form the α(1→6) branches of the glycogen.

Different branching enzymes of microbial origin have meanwhile been isolated and studied. US patent 4,454,161 discloses, e.g., a branching enzyme isolated from Bacillus megaterium. This enzyme is stable up to about 45°C and shows an optimum of enzyme activity at a temperature of about 25°C.

An article by Boyer and Preiss in Biochemistry 16, 1977, 3693-3699, discloses a branching enzyme from Escherichia coli. Baecker et al, J. Biol. Chem. 261, 1986, 8738-8743 disclose the nucleotide sequence of the glgB gene coding for this branching system, as well as the amino acid sequence of the enzyme corresponding therewith. This E. coli enzyme has a molecular weight of 84231 calculated from the amino acid composition.

Zevenhuizen, Biochim. Biophys. Acta 81, 1964, 608-611 describes a branching enzyme of Arthrobacter globiformis.

Walker and Builder, Eur. J. Biochem. 20, 1971, 14-21 describe a branching enzyme of Streptococcus mitis.

Steiner and Preiss, J. Bacteriol. 129, 1977, 246-253 describe a branching enzyme of Salmonella typhimurium.

Fredrick, J. Thermal Biol. 3, 1978, 1-4 and Phytochemistry 19, 1980, 539-542 describes a branching enzyme from the alga Cyanidium caldarium.

Kiel et al, Gene 78, 1989, 9-17 describe the cloning and expression in E. coli of the gene coding for a branching enzyme of Synechococcus sp. PCC 7942 (or Anacystis nidulans R2). The gene codes for two proteins the larger of which, as appears from the meanwhile determined nucleotide sequence of the glgB gene coding therefor, has a molecular weight of 89206. The enzyme activity of this branching enzyme shows an optimum at a temperature of about 35°C. The smaller protein has a molecular weight of approximately 72 kDa and is likewise enzymatically active.

As discussed in the above-mentioned US patent 4,454,161, branching enzyme can be used for improving the quality of different foods. The tendency of starch-like materials towards retrogradation leading to a decrease of the shelf life and digestibility of foods containing these materials can be suppressed by introducing α(1→6) branches by means of a branching enzyme.

It is a drawback of all the hitherto known branching enzymes that they are not very active and even unstable at elevated temperatures. This imposes substantial limitations on the method of treating a starch-like material to be modified or a product designed for human or animal consumption and containing a starch-like material to be modified. For different reasons a treatment at elevated temperatures, e.g., at a temperature above 45°C, may be preferred or even be necessary. If the treatment can be carried out at higher temperatures, e.g., higher starch concentrations can be used, which is desirable for many applications on a technical scale. This is not possible with the known branching enzymes.

Of different enzymes the existence of thermostable variants is known. Thus, for instance, European patent application EP-A-0 057 976 discloses a thermostable α-amylase enzyme originating from bacteria of a Bacillus stearothermophilus strain. This European patent application describes the cloning and expression of this thermostable α-amylase in E. coli and in B. subtilis. α-Amylase is an enzyme capable of being used for a hydrolytic degradation of starch-like materials, e.g., for the preparation of glucose syrup from corn starch.

Furthermore, US patent 4,612,287 discloses a thermostable pullulanase enzyme originating from the anaerobic microorganism Thermoanaerobium brockii. This US patent describes the cloning and expression of this thermostable pullulanase enzyme in E. coli and in B. subtilis. Pullulanase is an enzyme which specifically catalyzes the cleavage of α(1→6) glucosidic linkages and, therefore, leads to a decrease of the number of branches in amylopectin and similar starch materials.

Up to now, thermostable variants of the branching enzyme have not been described.

### Description of the invention

The invention meets the existing need for a thermostable branching enzyme and particularly provides a branching enzyme of microbial origin obtained by isolation from a microorganism capable of expressing the enzyme, which novel branching enzyme is characterized according to the invention by a stability of the enzyme at temperatures of at least 50°C and an optimum of the enzymatic activity at a temperature above 45°C.

A preferred embodiment of the invention relates to a branching enzyme characterized by an origin from bacteria of the species Bacillus stearothermophilus, more in particular by an origin from bacteria of the strain Bacillus stearothermophilus 1503-4R var.4.

Although the known thermophilic microorganism Bacillus stearothermophilus normally does not form appreciable amounts of glycogen, the above-mentioned spontaneous variant Bacillus stearothermophilus 1503-4R var.4 is capable of accumulating at the end of the logarithmic growth phase a branched glucan in which the average chain length of the branches is at 21 glucose residues. In articles published in Biochim. Biophys. Acta 177, 1969, 166-168 and in Arch. Biochem. Biophys. 149, 1972, 252-258, as well as in the book "Biochemistry of the glycosidic linkage", vol. 2, 1972, 621-627, Goldemberg has reported on the glucan biosynthesis of the above variant. However, the isolation, characterization and cloning of the responsible branching enzyme are not mentioned in these publications.

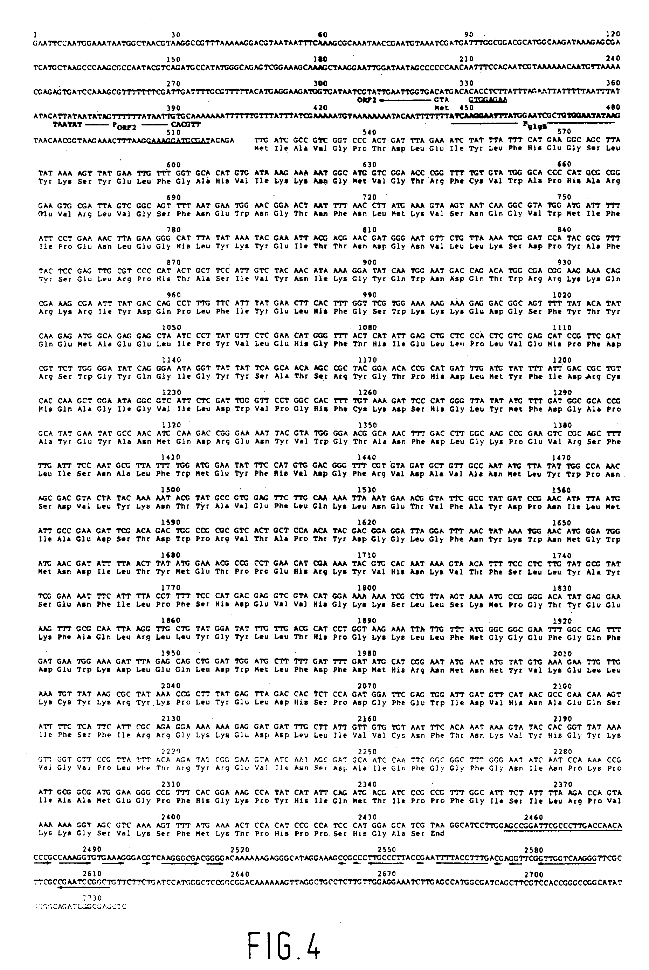

The present inventors have cloned, characterized and expressed in E. coli and in B. subtilis the glgB gene of Bacillus stearothermophilus 1503-4R var. 4 coding for its thermostable branching enzyme. From the nucleotide sequence determined by the inventors they could derive the amino acid sequence and from this they could calculate the molecular weight of the thermostable branching enzyme at 74787. The enzyme consists of 639 amino acid reidues (see Fig. 4). Furthermore, the inventors have studied the enzymatic activity of the relevant thermostable branching enzyme. They found that the enzyme is stable up to a temperature of at least 65°C and shows an optimum activity at a temperature of about 53°C.

Although according to the experimental part of this application the inventors have expressed the glgB gene of Bacillus stearothermophilus only in E. coli and in B. subtilis, the information supplied in the experimental part will be sufficient for a skilled worker to express the gene in other hosts too. As a rule, it will only be necessary to accommodate the regulatory elements and the transformation method, that is to say, e.g., it will be possible to realize expression in lactic acid bacteria by incorporating the structural glgG gene into an expression cassette useful for lactic acid bacteria and by using a vector suitable for the transformation of lactic acid bacteria. The use of other microorganisms can be preferred for different reasons, e.g., for reasons of safety (lactic acid bacteria belong to the so-called GRAS bacteria, which is an important advantage in food applications) or for reasons of productivity. The invention further renders it possible to realize a high production of the branching enzyme by using regulatory elements suitable for that purpose, such as a strong transcription promoter.

Of course, the invention is not limited to the branching enzyme originating from B. stearothermophilus and having the amino acid sequence shown in Fig. 4. Mutants of this enzyme having a different amino acid sequence are also comprised by the invention, provided they are active and meet the requirements imposed on stability and activity at higher temperatures. The same applies to corresponding enzymes originating from other microorganisms. As far as the gene coding for the thermostable branching enzyme is concerned, the invention is not limited to the nucleotide sequence shown in Fig. 4, but comprises mutants thereof which owing to the genetic code being degenerate code for an enzyme having the same amino acid sequence, as well as mutants coding for a mutant enzyme if the mutant enzyme meets the conditions set forth above, and in a broader sense any gene of microbial origin coding for a thermostable branching enzyme as herein defined. The invention comprises recombinant polynucelotides (preferably DNA, but recombinant RNA is also comprised) containing such a gene. Such recombinant polynucleotides may further comprise regulatory elements, such as a transcription promoter suitable for the selected host, and may also comprise a vector part and have the form of a chimeric plasmid comprising a vector plasmid containing an insertion of DNA comprising a transcription promoter and, if required, other regulatory elements, as well as DNA coding for a thermostable branching enzyme according to the invention.

The invention also extends to the use of the novel thermostable branching enzyme according to the invention in processes for the modification of a starch-like material such as starch, amylose, amylopectin, dextrin, and other polyglucose materials, and to the thus obtained modified starch-like material. Of course, modification means introduction of (additional) branches.

The invention further resides in a process for the preparation or manufacture of a product suitable for human or animal consumption (foods and fodders), which product comprises a starch-like material modified by treatment with a branching enzyme according to the invention.
The invention will now be explained further with reference to the accompanying drawings and the following experimental part.

### Description of the drawings

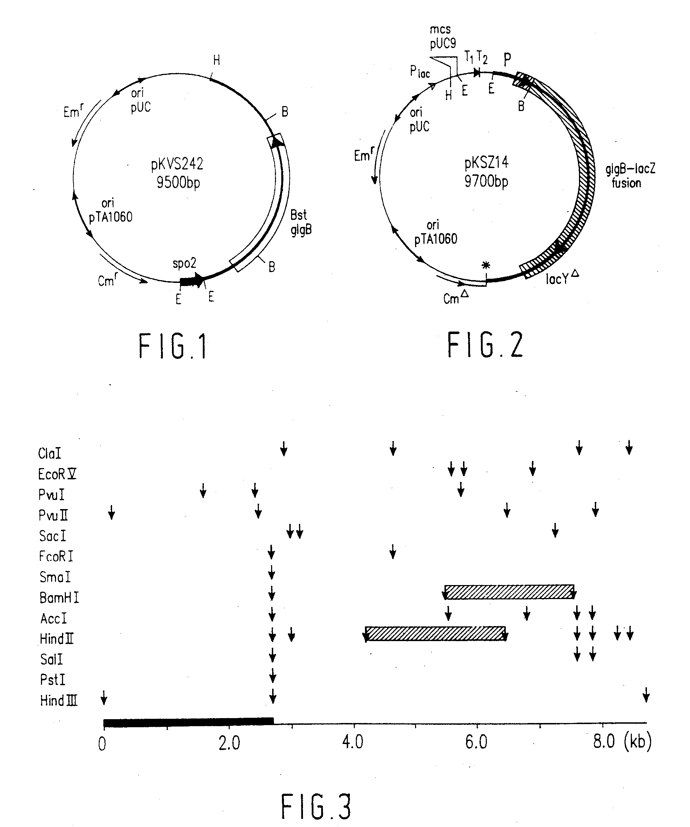

Fig. 1 shows the map of plasmid pKVS242 partly originating from the starting plasmid pHP13 (thin lines). Insertions are indicated by thick lines. The fat arrow represents the SPO2 promoter. The open block represents the coding region of the Bacillus stearothermophilus (Bst) glgB gene. The abbreviations have the usual meanings:
Em^{r} indicates the erythromycin resistance gene,
Cm^{r} indicates the chloramphenicol resistance gene,
ori indicates the replication origin,
E indicates an EcoRI site, B a BamHI site, and H a HindIII site.

Fig. 2 shows the map of plasmid pKSZ14, the hatched block representing the coding region of the glgB-lacZ fusion gene. For the used abbreviations it applies what has been observed for Fig. 1, and it further applies:
Δ indicates a blunt-ended gene,
mcs indicates a multiple cloning site,
P_{lac} indicates the location and orientation of the lacZ promoter,
P indicates the putative Bst glgB promoter,
* indicates the location of an NcoI-XmnI fusion,
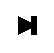 indicates the location and orientation of the T1T2 terminators.

Fig. 3 shows the restriction map of plasmid pKVS1. The thin line indicates the Bst insertion, and the thick line indicates the DNA originating from vector pUC9. The hatched areas indicate the fragments which most strongly hybridized with the E. coli glgB probe.

Fig. 4 shows the nucelotide sequence of the Bst glgB gene (the antisense strand of the 2.7 kb EcoRI-SacI fragment) and the amino acid sequence of the branching enzyme encoded by it. The open reading frame starting at nucleotide 325 is indicated as ORF2. Putative ribosome binding sites are underlined. Putative promoters are indicated by letters printed in heavy type. The arrows downstream from the Bst glgB gene indicate a region of dyad symmetry.

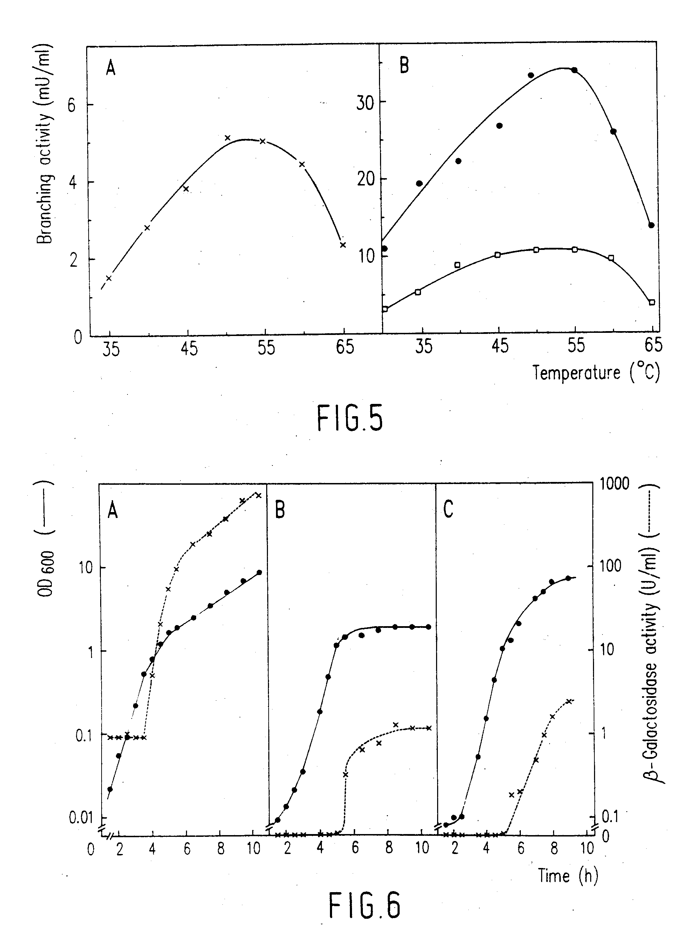

Fig. 5 shows the activity of the Bst branching enzyme and the effect of the temperature on that activity. The branching activity was determined in a DEAE-purified preparation of B. stearothermophilus 1503-4R var. 4 (crosses in pane, A) and in extracts of E. coli KV832[pKVS242] (open squares in panel B) and B. subtilis 5GM(amy) [pKVS242] (massive globules in panel B) by the increase in the number of 1,6-α-glucosidic linkages in amylopectin at the indicated temperatures. A unit of activity was defined as the amount of enzyme required for introducing per minute 1 µmol α-1,6-glucosidic linkages into the substrate.

Fig. 6 shows the expression of the Bst glgB promoter in B. subtilis. The growth curves (massive globules) and the formation of β-galactosidase activity through a correct glgB-lacZ fusion (crosses) for B. subtilis 8G5[pKSZ14] cultured on selective TY-nutrient medium (panel A) or cultured on a selective TY-nutrient medium supplemented with 0.5% glucose panel B), and for B. subtilis IS233[pKSZ14] cultured on selective TY-nutrient medium (panel C) are shown in this figure. The specific β-galactosidase activities are expressed in Miller units per ml sample. The time starts at the moment of dilution in fresh medium.

### Experimental part

### Cloning of the glgB gene of B. stearothermophilus

A cross-hybridization experiment was conducted in which, after cutting with HindIII and BamHI, DNA of B. stearothermophilus was hybridized at 58°C and 61°C with a 1.2 kb BamHi fragment serving as a probe. This 1.2 kb BamHI fragment was isolated from plasmid pOP190 and contains nucleotides 256-1464 of the E. coli glgB gene. The results of the Southern blot analysis are not shown here.

For cloning a HindIII fragment of about 6 kb was selected which showed a strong hybridization with the E. coli glgB probe and was sufficiently large to contain the Bst glgB gene (assuming that this gene is comparable in size to the glgB genes of E. coli and Synechococcus sp., which are respectively 2.2 and 2.3 kb). The Bst DNA HindIII fraction of 4-7 kb was isolated and cloned into the HindIII site of plasmid pUC9, followed by transformation into E. coli KV832. Recombinant colonies were transferred to nitrocellulose filters and hybridized at 58°C using the 1.2 kb BamHI fragment of the E. coli glgB gene as a probe. Since this fragment is deleted in the chromosome of E. coli KV832, only clones will hybridize that carry Bst insertions showing similarity in sequence with the E. coli glgB probe. Positive colonies were analyzed by manufacturing restriction maps and were tested by additional cross-hybridization experiments. A plasmid containing a 6.1 kb HindIII fragment showing a strong hybridization with the E. coli glgB probe was selected and indicated as pKVS1.

### Characterization of pKVS1

Fig. 3 shows the restriction map of pKVS1. Also indicated are the restriction fragments of the Bst insertion hybridizing with the E. coli probe. Plasmid pKVS1 gave no expression of the Bst glgB gene, as was concluded from its inability of complementation of E. coli glgB. In a complementation experiment with plasmid pKVS2 (a derivative of pKVS1 in which the Bst insertion is cloned into pUC9 in opposite orientation) the colonies turned brown, however, after dyeing with iodine, which indicates the presence of a branched polyglucose. This result shows that the pUC9 carries a DNA fragment coding for a branching enzyme.

### Nucleotide sequence of the Bst glgB gene

The nucleotide sequence of a 2.7 kb EcoRI-SacI fragment containing the cloned gene and the flanking regions was determined. Analysis of the sequence shown in Fig. 4 exhibits an open reading frame of 1917 nucleotides coding for a polypeptide of 639 amino acids. A Shine-Dalgarno-like sequence (underlined in Fig. 4) is located upstream from the initiation triplet TTG. The strength of the interaction between this putative S.D. sequence and the 3′ end of the gram-positive 16S rRNA was calculated according to Tinoco et al, Nature 246, 1973, 40-41 at ΔG=-15.2 kcal/mol, which is a value normal to gram-positive ribosome binding sites.

Upstream from the Bst glgB gene no sequences were found showing similarity to the consensus -35 and -10 regions recognized by the most important vegetative B. subtilis RNA polymerase E-σ^{A}. However, a strong homology was observed between the region preceding the S.D. sequence (in Fig. 4 this region is indicated by letters printed in heavy type) and the consensus sequence for promoters recognized by the B. subtilis RNA polymerase E-σ^{H} containing sigma factor H.

Further upstream from thc coding region of the Bst glgB gene the N-terminal part of a second open reading frame is present. This open reading frame designated ORF2 in Fig. 4 extends to the EcoRI site (nucleotide 1 of the sequence). No similarities were found between the partial amino acid sequence of ORF2 and known amino acid sequences. Located upstream from the ATG initiation codon of ORF2 is a putative S.D. sequence (underlined in Fig. 4) with a ΔG for binding to the 3′ end of the gram-positive 16S rRNA of -14.2 kcal/mol. Furthermore, upstream from this S.D. sequence a sequence indicated in Fig. 4 by letters printed in heavy type is present resembling the -35 and -10 regions recognized by E-σ^{A}. Interposed between the two putative promoters is an A/T rich region.

Eleven nucleotides downstream from the translational termination codon TAA of the Bst glgB gene an extended region of dyad symmetry is present. Upon transcription into RNA this region can form a stem and loop structure with a ΔG of -104 kcal/mol. This region does not appear to be a rho-independent transcriptional termination site, since it lacks a thymine-rich region immediately downstream from the stem and loop structure.

### Amino acid sequence of the Bst branching system

From the amino acid sequence shown in Fig. 4 it appears that the Bst branching system has a molecular weight of 74787, i.e. substantially below the molecular weights of the known branching enzymes of E. coli (Ec) and Synechococcus sp. (An), which are respectively 84 and 89 kDa. A comparison of the amino acid sequences of these branching systems reveals that the gram-positive branching enzyme lacks the N-terminal of the gram-negative enzymes. Although the amino acid sequences of the Bst, Ec and An branching enzymes in the central part of the proteins show great similarity, the total homology resulting from the very limited similarity in the N-terminal part is relatively poor.

### Codon use and G+C content of the Bst glgB gene

A comparison of the codon use in the above glgB genes reveals great differences between the gram-positive and gram-negative genes. In the gram-negative genes rare codons such as TTA, ATA, GTA, ACA, AAA, CGA, AGA, and GGA are often used in the Bst glgB gene. It further appears that in the gram-positive glgB gene there is a strong resistance to G and C at all positions, but especially at the third position of the codon. In the gram-negative genes, however, there is a clear preference for C and resistance to A at the third position of the codons. The G+C content of the Bst glgB gene (40.8%) is therefore remarkably much lower than that of the gram-negative Ec and An glgB genes (respectively 53.2 and 56.5%).

### Amino acid composition

Also in the amino acid composition there prove to be rather great differences between the above branching enzymes. Thus the Bst branching enzyme has a much lower arginine and a much higher lysine content than the branching enzymes of the gram-negative bacteria, and the gram-positive enzyme contains a much greater number of hydrophobic amino acids (Phe, Trp, Tyr, Ile, Leu, Met and Val), namely 39.3% versus only 34.9% in the enzymes of the gram-negative bacteria.

### Expression of the Bst glgB gene in E. coli and B. subtilis

Expression of the Bst branching enzyme in E. coli transformed with pKVS2 was rather weak. A shuttle plasmid pKVS242 was constructed to obtain a stronger expression in E. coli and also to obtain expression in B. subtilis. This plasmid shown in Fig. 1 was constructed by ligating a 4.3 kb EcoRI-HindIII fragment cut from pKVS1 and containing the complete Bst glgB gene in pHP13 cut with the same restriction enzymes and then inserting the very strong B. subtilis phage SPO2 promoter (Williams et al, J. Bacteriol. 146, 1981, 1162-1165) as a 280 bp EcoRI fragment into the unique EcoRI site. This 280 bp EcoRI fragment was isolated from plasmid pGKV21 described by van der Vossen et al in Appl. Environ. Microbiol. 50, 1985, 540-542. Its ability of complementation of E. coli KV832 showed that pKVS242 is capable of expressing the Bst glgB gene.

Extracts were prepared from pKVS242 containing bacteria of the bacterial strains E. coli KV832 and B. subtilis 5GM(amy). The activity and the effect of the temperature on the activity of the branching enzyme in these extracts were determined and compared with a DEAE-purified enzyme preparation of Bst 1503-4R var. 4. The results shown in Fig. 5 indicate that the number of α-1,6-glucosidic linkages in amylopectin is efficiently increased by extracts from pKVS242-containing cells. In control tests with extracts from KV832[pHP13] and 5GM(amy)[pHP13} values below 1 milli unit per ml were measured. Furthermore, the activity in KV832[pKVS2] was just above the background (data not shown). Fig. 5 also shows that in all cases the optimum branching activity was obtained at about 53°C, which proves the thermostable nature of the cloned enzyme.

### Growth phase dependent expression in B. subtilis

In order to test whether transcription of the Bst glgB gene takes place when using a promoter recognized by E-σ^{H}, plasmid pKSZ14 shown in Fig. 2 and containing a glgB-lacZ fusion gene (with a correct reading frame) was constructed. The plasmid was constructed from 4 fragments, namely an 840 bp EcoRI-BamHI fragment isolated from pKVS1 and containing the 5′ end of the Bst glgB gene including the putative transcription and translational regulatory elements; the B. subtilis/E. coli shuttle vector pHP13 cut with NcoI (blunted) and EcoRI; a 4.3 kb BamHi-XmnI fragment cut from pMLB1034 and containing a blunt-ended lacZ gene; and a 500 bp EcoRI fragment containing the E. coli rrnB T1T2 terminators (Brosius, Gene 27, 1984, 161-172). The glgB-lacZ fusion gene codes for a fusion protein having β-galactosidase activity. Furthermore, the T1T2 terminator stops transcription starting upstream from the fusion gene at the lacZ promoter and hindering accurate determination of the activity of the putative Bst glgB promoter. Upon transformation of B. subtilis 8G5 with pKSZ14 blue colonies were obtained after prolonged growth on XGal-containing selective TY agar plates.

Panel A of Fig. 6 shows the expression pattern of the β-galactosidase activity in B. subtilis 8G5[pKSZ14]. During vegetative growth no increase in the activity was observed. Apparently, the promoter of the Bst glgB gene was ruled out in this growth phase (the measured β-galactosidase activity is probably the residual activity of the overnight culture used as inoculum). At the end of the exponential growth phase the β-galactosidase activity rapidly increased, which indicates that the gene is expressed in the later growth stages. However, when the cells were cultured in medium supplemented with glucose, hardly any activity could be detected (see Panel B in Fig. 6). Although the expression pattern of β-galactosidase remained the same, a 500-fold decrease of the activity was observed. These results confirm that the Bst glgB gene is not expressed by E-σ^{A}, but that one or more of the minor sigma factors are involved in the expression of the gene in B. subtilis. Dependence on E-σ^{H} was tested by bringing plasmid pKSZ14 into B. subtilis IS233, which strain lacks sigma factor H. The results shown in panel C of Fig. 6 exhibit that very little β-galactosidase activity is obtained. Apparently, therefore, sigma factor H is actually involved in the expression of the Bst glgB gene in B. subtilis.

### Bacteria, plasmids, media and (bio)chemicals

The used bacterial strains and plasmids are given below. The B. subtilis strain 5GM(amy) mentioned below was obtained by congression after transformation of competent 6GM cells with DNA of B. subtilis strain 1-85(amy) and selection for tyr⁺ recombinants.

### B. subtilis

- 85: trpC2 met his ura rib tyr nic purA
Bron and Venema, Mutation Res. 15, 1972, 1-10
- 6GM: trpC2 met his ura rib tyr r_{M}⁻ m_{M}⁺
Haima et al, Mol. Gen. Genet. 209, 1987, 335-342
- 5GM(amy): trpC2 met his ura rib amy r_{M}⁻ m_{M}⁺
see the above description
- 1-85: trpC2 amy
Yuki, J. Genet. 42, 1967, 251-261
- IS233: trpC2 phe-1 spoOHΔHind
Weir et al, J. Bacteriol. 157, 1984, 405-412

### B. stearothermophilus

- 1503-4R: variant of B. stearothermophilus 1503-4R accumulating
- var.4: glucan
Goldemberg and Algranati, Biochim. Biophys. Acta 177, 1969, 166-168

### E. coli

- KV832: F⁻ ara Δ(lac-pro) thi strA Φ80dlacZΔM15 ΔglgB1200: :Km^{r} Kiel et al, Mol. Gen. Genet. 207, 1987, 294-301
- JM101: supE thi Δ (lac-proAB⁻) [F′traD36 proAB⁺ lacI^{q} lacZΔM15] Messing, Recombinant DNA Technical Bulletin, NIH publ. No. 79-99, vol. 2, No. 2, 1979, 43-48

### plasmids

- pUC9: Ap^{r}, 2.7 kb, E. coli replicon Vieira and Messing, Gene 19, 1982, 259-268
- pHP13: Em^{r} Cm^{r}, 4.9 kb, E. coli and B. subtilis replicons Haima et al, Mol. Gen. Genet. 209, 1987, 335-342
- pMLB1034: Ap^{r}, 6.3 kb, E. coli replicon, blunt-ended lacZ gene Silhavy et al, Experiments with gene fusions, 1984
- pOP190: Tc^{r}, 11.5 kb, E. coli replicon, E. coli asd and glgB genes
Kiel et al, Mol. Gen. Genet. 207, 1987, 294-301
- pKVS1: see earlier description in experimental part
- pKVS2: see earlier description in experimental part
- pKVS242: see earlier description in experimental part
- pKSZ14: see earlier description in experimental part

Medium 2 contained per liter 10 g casitone, 1 g yeast extract, 3 g glucose, 1.5 g K₂HPO₄, 1 g KH₂PO₄, 8 g NaCl, 0.04 g CaCl₂.2H₂O, 0.2 g MgSO₄, and 0.02 g FeCl₃.6H₂O pH 7.2. A TY nutrient medium contained per liter 10 g tryptone, 5 g yeast extract, 5 g NaCl, and 20 mg MnCl₂.4H₂O pH 7.4. If necessary, 0.5% glucose, 1.5% agar, or 40 µg/ml XGal (5-bromo-4-chloro-3-indolyl-β-D-galactoside) were added. As required, the antibiotics were supplemented: for E. coli Ap 100 µg/ml, Cm 10 µg/ml, Em 150 µg/ml, Km 50 µg/ml, and To 10 µg/ml; for B. subtilis Cm 5 µg/ml and Em 1 µg/ml. The media and procedures used for transformation of competent cells of B. subtilis were as described by Bron and Venema, Mutation Res. 15, 1972, 1-10. The restriction enzymes, T4 DNA ligase and Klenow polymerase originated from Boehringer, Mannheim, FRG, and they were used according to the instructions of the manufacturers. All other chemicals were of analytical grade.

### DNA preparations

Total DNA was isolated from E. coli JM101 cultured on TY nutrient medium at 37°C, and from B. stearothermophilus 1503-4R var. 4 cultured on TY nutrient medium at 55°C, supplemented with 0.5% glucose and 0.38 g CaCl₂, as described by Kiel et al, Mol. Gen. Genet. 207, 1987, 294-301. Preparative amounts of plasmid DNA were obtained from E. coli according to the alkaline lysis procedure of Maniatis et al, Molecular Cloning: A Laboratory Manual, 1982. The analytical "miniprep" procedure of Ish-Horowicz and Burke, Nucleic Acids Res. 9, 1981, 2989-2998, was used to extract plasmid DNA from 2 ml cultures of E. coli and 5 ml cultures of B. subtilis.

### Molecular cloning procedures

Routine DNA manipulations were performed as described by Maniatis et al. DNA restriction fragments were isolated from agarose gels with NA-45 DEAE membrane filters according to the instructions of the manufacturer (Schleicher and Schuell, Dassel, FRG).

### Cross-hybridization

Southern blot transfer was carried out by electroblotting of DNA restriction fragments on Genescreen plus filters according to the instructions of the manufacturer (Dupont, NEN, Boston, USA). Colony blotting was performed on nitrocellulose filters (Schleicher and Schuell, Dassel, FRG) as described by Maniatis et al. The filters were hybridized for at least 40 hours at 58°C or 61°C with [α-³²P]dCTP (3000 Ci/mmol, Radiochemical Centre, Amersham, UK) labelled probes in 5x SSC; 1% SDS; 0.02% polyvinylpyrrolidone; 0.02% bovine serum albumin; 0.02% Ficoll 400 and 250 µg/ml calf thymus DNA. After hybridization the filters were washed three times with 5x SSC at the hybridization temperature; 0.5% SDS for 1 hour. The filters were then air-dried and exposed to Kodak Omat films (XR-1).

### Determinations of branching enzyme activity.

Complementation analysis was used for the detection of plasma-encoded branching activity in E. coli KV832, as described by Kiel et al, Gene 78, 1989, 9-17. For the detection of branching activity in B. stearothermophilus, in E. coli, and in B. subtilis the following procedure was followed: B. stearothermophilus 1503-4R var. 4 cells were cultured in medium 2 to early stationary phase and then harvested by centrifugation. The cell pellet was suspended in 10 mM Tris-Cl pH 7.5, 1 mM EDTA, 1 mM DTT (buffer A) corresponding to a concentration of 5% dry weight, and the cells were disrupted by using an ultrasonic source (MSE Ltd.) in 20 min with pulses of 60 sec and intervals of 30 sec. Cell debris was removed by centrifugation. Then fractionation with (NH₄)₂SO₄ was performed. The material that precipitated between 20% and 55% saturation was pelleted, dissolved in buffer A and dialyzed extensively against the same buffer. Subsequently, the extract was loaded on a DEAE-cellulose column (40 ml, Whatman DE 52) in buffer A. The column was then eluted with a linear salt gradient (from 0 to 0.675 M NaCl in buffer A). The branching activity eluted at 0.35 M NaCl and was substantially free from amylase activity. Extracts from transformed E. coli KV832 and B. subtilis 5GM(amy) cells cultured on a selective TY nutrient medium supplemented with 0.5% glucose were prepared as described by Kiel et al, Gene 78, 1989, 9-17. The branching activity was determined in these extracts by the procedure described in the same publication, allowing a quantitative measurement of α-1,6-glucosidic linkages, but the substrate concentration was increased to 1.0% amylopectin.

### Nucleotide sequence analysis

DNA restriction fragments were subcloned into M13mp18 and M13mp19 (Norrander et al, Gene 26, 1983, 101-106) and transformed into E. coli JM101. Nucleotide sequences were determined by the dideoxy nucleotide chain-termination method of Sanger et al, PNAS USA 74, 1977, 5463-5467, using the Klenow fragment of DNA polymerase I, the universal sequence primer and [³⁵S]dATPαS (Biggin et al, PNAS USA 80, 1983, 3963-3965).

### Determination of the β-galactosidase activity.

Cells were cultured overnight at 37°C in 10 ml selective TY nutrient medium or selective TY nutrient medium supplemented with 0.5% glucose. The cultures were then diluted 1000-fold in 15 ml fresh medium. The cells were cultured at 37°C with vigorous agitation. Every 30-60 minutes samples of 0.1-1.0 ml were taken, if necessary diluted in TY nutrient medium, followed by determining the OD₆₀₀. Simultaneously, a sample of 0.2 ml was taken and stored overnight at -20°C. After thawing the bacteria were made permeable with chloroform and SDS, and the β-galactosidase activity was determined with o-nitrophenyl-β-D-galactopyranoside as a substrate according to the method of Miller, 1972. The activities, not corrected for OD₆₀₀ values and therefore representing rhe activity per ml culture, are expressed in Miller units.

## Claims

1. A branching enzyme of microbial origin obtained by isolation from an organism capable of expressing the enzyme, characterized in that the enzyme originates from bacteria of the strain Bacillus stearothermophilus 1503-4R var.4, has a stability at temperatures of at least 50°C and has an optimum of the enzymatic activity at a temperature above 45°C.

2. A branching enzyme according to claim 1, characterized in that the enzyme has the amino acid sequence shown in Fig. 4.

3. A branching enzyme according to claim 1, characterized in that the enzyme has been isolated from bacteria which, by genetic engineering by means of a recombinant polynucleotide, have been provided with genetic information necessary for expression of the enzyme.

4. A process for modifying a starch-like material by treating it with a branching enzyme of microbial origin obtained by isolation from an organism capable of expressing the enzyme, characterized by using a branching enzyme according to any of claims 1-3.

5. A recombinant nucleotide, in particular recombinant DNA, characterized in that it contains the genetic information coding for a branching enzyme according to any of claims 1-3.

6. A recombinant polynucleotide according to claim 5, characterized in that it comprises the nucleotide sequence coding for branching enzyme of bacteria of the strain Bacillus stearothermophilus 1503-4R var.4, as shown in Fig. 4.

7. A recombinant polynucleotide according to claim 5 or 6, characterized in that it further comprises a transcription promoter and, if required, other regulatory elements.

8. A chimeric plasmid comprising a vector plasmid containing an insertion of DNA comprising a transcription promoter and, if required, other regulatory elements, characterized in that the DNA insertion also comprises DNA coding for a branching enzyme according to any of claims 1-3.

9. A chimeric plasmid according to claim 8, characterized in that the DNA insertion comprises the nucleotide sequence coding for branching enzyme of bacteria of the strain Bacillus stearothermophilus 1503-4R var.4,.as shown in Fig. 4.

## Patentansprüche

1. Verzweigungsenzym mikrobiologischen Ursprungs, erhalten durch Isolierung aus einem Organismus, der in der Lage ist, das Enzym zu exprimieren, dadurch gekennzeichnet, daß das Enzym von Bakterien des Stammes Bacillus stearothermophilus 1503-4R var.4 stammt, eine Stabilität bei Temperaturen von mindestens 50°C besitzt und ein Optimum der enzymatischen Aktivität bei einer Temperatur über 45°C aufweist.

2. Verzweigungsenzym nach Anspruch 1, dadurch gekennzeichnet, daß das Enzym die in Figur 4 gezeigte Aminosäurensequenz aufweist.

3. Verzweigungsenzym nach Anspruch 1, dadurch gekennzeichnet, daß das Enzym aus Bakterien isoliert wurde, die über gentechnologische Maßnahmen mit Hilfe eines rekombinierten Polynucleotides mit zum Exprimieren des Enzyms erforderlichen genetischen Informationen versehen worden sind.

4. Verfahren zum Modifizieren eines stärkeähnlichen Materiales durch Behandeln des Materiales mit einem Verzweigungsenzym mikrobiologischen Ursprungs, das durch Isolierung aus einem Organismus erhalten wurde, der zum Exprimieren des Enzyms in der Lage ist, gekennzeichnet durch Verwendung eines Verzweigungsenzyms nach einem der Ansprüche 1 - 3.

5. Rekombiniertes Nucleotid, insbesondere rekombinierte DNA, dadurch gekennzeichnet, daß es den genetischen Informationscode für ein Verzweigungsenzym nach einem der Ansprüche 1 - 3 enthält.

6. Rekombiniertes Polynucleotid gemäß Anspruch 5, dadurch gekennzeichnet, daß es den Nucleotidsequenzcode für das Verzweigungsenzym aus Bakterien des Stammes Bacillus stearothermophilus 1503-4R var.4, wie in Figur 4 gezeigt, enthält.

7. Rekombiniertes Polynucleotid nach Anspruch 5 oder 6, dadurch gekennzeichnet, daß es des weiteren einen Transkriptions-Promotor und, falls erforderlich, andere regulierende Elemente enthält.

8. Heterozygotes Plasmid mit einem Vektorplasmid, das eine Insertion von DNA, die einen Transkriptions-Promotor und, falls erforderlich, andere regulierende Elemente aufweist, umfaßt, dadurch gekennzeichnet, daß die DNA-Insertion auch einen DNA-Code für ein Verzweigungsenzym nach einem der Ansprüche 1 - 3 enthält.

9. Heterozygotes Plasmid nach Anspruch 8, dadurch gekennzeichnet, daß die DNA-Insertion den Nucleotidsequenzcode für das Verzweigungsenzym aus Bakterien des Stammes Bacillus stearothermophilus 1503-4R var.4, wie in Figur 4 gezeigt, enthält.

## Revendications

1. Enzyme branchante d'origine microbienne obtenue par isolement à partir d'un organisme capable d'exprimer l'enzyme, caractérisée en ce que l'enzyme prend son origine dans une bactérie de la souche 1503-4R var. 4 de Bacillus stéarothermophilus, est stable à des températures d'au moins 50°C et a un optimum d'activité enzymatique au niveau d'une température située au-dessus de 45°C.

2. Enzyme branchante salon la revendication 1, caractérisée en ce que l'enzyme comporte la séquence d'acides aminés représentée sur la figure 4.

3. Enzyme branchante selon la revendication 1, caractérisée en ce que l'enzyme a été isolée à partir d'une bactérie qui, par manipulation génétique à l'aide d'un polynucléotide recombinant, a été munie d'une information génétique nécessaire pour l'expression de l'anzyma.

4. Procédé pour modifier un matériel analogue à l'amidon en le traitant avec une enzyme branchante d'origine microbienne obtenue par isolemant à partir d'un orgarisme capable d'exprimer l'enzyme, caractérisé en ce qu'on utilise une enzyme branchante selon l'une quelconque des revendications 1 à 3.

5. Nucléotide recombinant, en particulier de l'ADN recombinant, caractérisé en ce qu'il comporte l'information génétique codant pour une enzyme branchante selon l'une quelconque de revendications 1 à 3.

6. Polynucléotide recombinant selon la revendication 5, caractérisé en ce qu'il comporte la séquence nucléotidique codant pour une enzyme branchante d'une bactérie de la souche 1503-4R var. 4 de Bacillus stéarothermophilus, telle que représentée sur la figure 4.

7. Polynucléotide recombinant selon la revendication 5 ou 6, caractérisé en ce qu'il comporte en outre un promoteur de la transcription et si nécessaire, d'autres éléments régulateurs.

8. Plasmide chimérique comporant un plasmide vecteur contenant une insertion d'ADN comportant un promoteur de transcription et si nécessaire d'autres éléments régulateurs, caractérisé en ce que l'insertion d'ADN comporte aussi l'ADN codant pour une enzyme branchante selon l'une quelconque des revendications 1 à 3.

9. Plasmide chimérique selon la revendication 8, caractérisé en ce que l'insertion d'ADN comporte la séquence nucléotidique codant pour l'enzyme branchante d'une bactérie de la souche 1503-4R var. 4 Bacillus stéarothermophilus, telle que représentée sur la figure 4.
